# EUROPÄISCHE PATENTSCHRIFT

(11) **EP 0 373 104 B1**
(45) Veröffentlichungstag und Bekanntmachung des Hinweises auf die Patenterteilung: **13.07.1994**
(21) Anmeldenummer: 89810848.5
(22) Anmeldetag: 08.11.1989
(51) Int. Cl.: C07D 493/10, C09B 11/08

(54) **Veretherte Fluoresceinverbindungen**
Etherified fluorescein compounds
Composés éthérifiés de fluorescéine

(30) Priorität: 17.11.1988 CH 4259/88
(43) Veröffentlichungstag der Anmeldung: 13.06.1990
(73) Patentinhaber: CIBA-GEIGY AG, 4002 Basel (CH)
(72) Erfinder: Wietfeld, Bernhard, Dr., D-7859 Efringen-Kirchen 2 (DE)

(56) Entgegenhaltungen:
- FR-A- 2 216 278
- JOURNAL OF THE AMERICAN CHEMICAL SOCIETY, Band 110, Nr. 1, 6. Januar 1988, Seiten 301-303, American Chemical Society; G.A. KRAFFT et al.: "Photoactivable fluorophores. 3. Synthesis and photoactivation of fluorogenic difunctionalized fluoresceins 1,2"
- CHEMICAL ABSTRACTS, Band 108, Nr. 20, 16. Mai 1988, Seite 640, ZusammenfassungNr. 177032m, Columbus, Ohio, US

## Beschreibung

Die vorliegende Erfindung betrifft neue veretherte Fluoresceinverbindungen, Verfahren zu ihrer Herstellung und ihre Verwendung als Farbbildner in Aufzeichnungsmaterialien.

In der FR-A-2,216,278 werden Dilactonverbindungen offenbart, die in druckempfindlichen Aufzeichnungsmaterialien Verwendung finden.

3,6-Bis-n-butoxyfluoran, das in Lösungen eingekapselt wird, ist in Chem. Abstr., Band 108, Nr. 20,1988 beschrieben.

Die erfindungsgemässen veretherten Fluoresceinverbindungen entsprechen der allgemeinen Formel 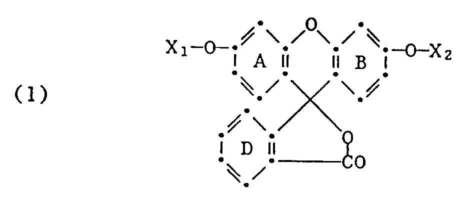
worin
X₁ Alkyl mit 4 bis 12 Kohlenstoffatomen oder C₅-C₆-Cycloalkyl und
X₂ unsubstituiertes oder durch Halogen, Hydroxy, Cyano oder C₁-C₅-Alkoxy substituiertes Alkyl mit 1 bis 12 Kohlenstoffatomen oder C₅-C₆-Cycloalkyl bedeuten, die Ringe A und B, unabhängig voneinander, unsubstituiert oder durch Halogen, C₁-C₅-Alkyl oder C₁-C₅-Alkoxy substituiert sind und der Ring D unsubstituiert oder durch Halogen, Nitro, C₁-C₅-Alkyl, C₁-C₅-Alkoxy, C₁-C₅-Alkylthio, C₁-C₅-Alkoxycarbonyl, Amino, Mono C₁-C₅-Alkylamino oder Di C₁-C₅-Alkylamino substituiert ist, mit der Ausnahmne, dass X₁ und X₂ nicht gleichzeitig n-Butyl darstellen.

Beispiele für C₁-C₅-Gruppen sind Methyl, Ethyl, n-Propyl, Isopropyl, n-Butyl, sek.-Butyl, Isobutyl, tert.Butyl, Amyl, Isoamyl oder sek.Amyl bzw. Methoxy, Ethoxy, Isopropoxy, Isobutoxy oder tert.Butoxy bzw. Methylthio, Ethylthio, Propylthio oder Butylthio.

Halogen bedeutet beispielsweise Fluor, Brom oder vorzugsweise Chlor.

Als Alkylreste können X₁ und X₂ geradkettig oder verzweigt sein. Beispiele solcher Alkylreste sind n-Butyl, sek. Butyl, Isobutyl, 1,1,3,3-Tetramethylbutyl, Amyl, Isoamyl, n-Hexyl, 2-Ethylhexyl, n-Heptyl, n-Octyl, Isooctyl, n-Nonyl, Isononyl oder n-Dodecyl. X₂ kann auch Methyl, Ethyl, n-Propyl oder Isopropyl sein.

Ist der Alkylrest in X₂ substituiert, so handelt es sich um Cyanoalkyl, Halogenalkyl, Hydroxyalkyl, Alkoxyalkyl jeweils vorzugsweise mit insgesamt 2 bis 8 Kohlenstoffatomen, wie z.B. 2-Cyanoethyl, 2-Chlorethyl, 3-Chlorethyl, 2-Hydroxyethyl, 3-Hydroxypropyl, 2-Methoxyethyl, 2-Ethoxyethyl oder 3-Methoxypropyl.

Beispiele für Cycloalkyl in der Bedeutung der X-Reste sind Cyclopentyl oder vorzugsweise Cyclohexyl.
- X₁: ist vorzugsweise n-Butyl, Isobutyl, sek. Butyl, 1,1,3,3-Tetramethylbutyl, n-Pentyl, Isopentyl, n-Hexyl oder n-Octyl.
- X₂: ist vorzugsweise C₁-C₈-Alkyl, 2-Cyanoethyl, 2-Methoxyethyl oder 2-Ethoxyethyl und besonders Methyl, Ethyl, n-Propyl, Isopropyl, n-Butyl, sek. Butyl, 1,1,3,3-Tetramethylbutyl, Isopentyl oder n-Octyl.

Die Ringe A und B sind vorteilhafterweise nicht weiter substituiert. Falls die Ringe A und B substituiert sind, so tragen sie vorzugsweise, unabhängig voneinander, jeweils 1 oder 2 Halogenatome.

Der Ring D kann in erster Linie durch Halogen, Nitro, C₁-C₅-Alkyl, C₁-C₅-Alkoxycarbonyl oder Di C₁-C₅-Alkylamino substituiert sein. Zweckmässigerweise ist der Ring D unsubstituiert.

Praktisch wichtige veretherte Fluoresceinverbindungen entsprechen der Formel 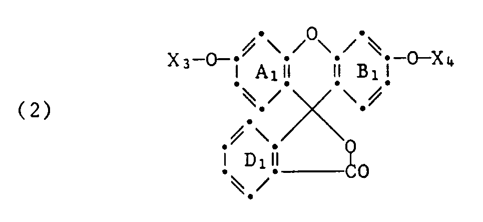
worin
- X₃: C₄-C₈-Alkyl und
- X₄: C₁-C₈-Alkyl oder durch Halogen, Cyano oder Niederalkoxy substituiertes C₂-C₄-Alkyl bedeuten und
die Ringe A₁, B₁ und D₁, unabhängig voneinander, unsubstituiert oder durch Halogen substituiert sind.

Unter den Laktonverbindungen der Formel (2) sind diejenigen, in denen
- X₄: C₁-C₅-Alkyl bedeutet, die Ringe A₁, B₁ und D₁ unsubstituiert sind, besonders bevorzugt.

Von besonderem Interesse sind veretherte Fluoresceinverbindungen der Formel 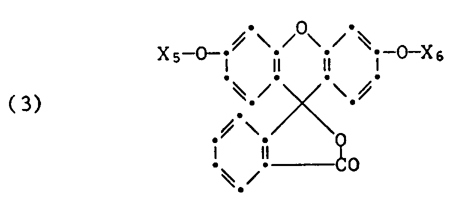
worin
- X₅: n-Butyl, sek. Butyl, Isobutyl, n-Pentyl, Isopentyl, Hexyl oder n-Octyl und
- X₆: Methyl, Ethyl, n-Propyl, Isopropyl, n-Butyl, sek. Butyl, n-Pentyl oder Isopentyl
bedeuten.

Sowohl in Formel (2) als auch in Formel (3) sind veretherte Fluoresceinverbindungen, in denen die X-Reste gleichzeitig n-Butyl sind, ausgeschlossen.

Die erfindungsgemässen veretherten Fluoresceinverbindungen werden dadurch hergestellt, dass man eine Fluoresceinverbindung der Formel 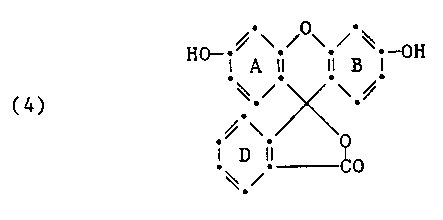
worin A, B und D die angegebene Bedeutung haben, mit einem entsprechenden Alkylierungsmittel bzw. Cycloalkylierungsmittel verethert.

Die Veretherung erfolgt zweckmässigerweise in Anwesenheit eines säurebindenden Mittels, wie z.B. eines Alkalimetallhydroxides, Alkalimetallcarbonates, einer tertiären Stickstoffbase, wie z.B. Pyridin oder Trialkylaminen, und vorzugsweise in Gegenwart auch eines quaternären Ammoniumsalzes, wie z.B. Tetrabutylammoniumbromides, gegebenenfalls in einem organischen Lösungsmittel oder in einem wässerig-organischen zweiphasigen Medium und bei Rückflusstemperatur.

Als Lösungsmittel kommen beispielsweise cycloaliphatische oder aromatisch Kohlenwasserstoffe, wie z.B. Cyclohexan, Benzol, Toluol oder Xylol; Chlorkohlenwasserstoffe, wie z.B. Chloroform, Ethylendichlorid oder Chlorbenzole insbesondere Dichlorbenzol; Ether, wie z.B. Diethylether oder Glykoldimethylether; cyclische Ether, wie z.B. Dioxan oder Tetrahydrofuran; sowie Dimethylformamid, Diethylformamid, Dimethylsulfoxid, Aceton oder Acetonitril in Betracht.

Sind X₁ und X₂ voneinander verschieden, so erfolgt die Veretherung mit den entsprechenden Alkylierungsmitteln in zwei Stufen, wobei X₁ und X₂ nacheinander eingeführt werden und die Zwischenprodukte gewünschtenfalls isoliert werden können. Werden hingegen X₁ und X₂ gleichzeitig eingeführt, so entstehen zweckmässigerweise Gemische der entsprechenden veretherten Fluoresceinverbindungen.

Die Isolierung der Endprodukte oder der gewünschten Zwischenprodukte erfolgt in allgemein bekannter Weise durch Behandeln der Reaktionprodukte mit geeigneten vorzugsweise nicht-polaren organischen Lösungsmitteln, wie z.B. Benzol, Chlorbenzol, Toluol oder Xylol, Trennung der entstandenen Phasen und Entfernung des Lösungsmittels aus den organischen Phasen. Nötigenfalls können die Produkte durch Umkristallisation z.B. aus Isopropanol gereinigt werden.

Geeignete Alkylierungsmittel sind Alkylhalogenide oder in manchen Fällen auch Dialkylsulphate wie Dimethyl- oder Diethylsulfat oder Tosylester, wie Tosylmethyl- oder Tosyl-n-butylester.

Zu spezifischen Beispielen für die Alkylhalogenide gehören Methylchlorid, Methylbromid, Methyljodid, Ethylchlorid, Ethylbromid, Ethyljodid, Propylchlorid, Isopropylchlorid, Propylbromid, n-Butylbromid, sek-Butylbromid, 1,1,3,3-Tetramethylbutylbromid, Amylbromid, Isopentylbromid, Hexylbromid, Heptylbromid, n-Octylbromid, 2,2-Dimethylpropylbromid, 1-Brom-2-chlorpropan, 1-Brom-3-chlorpropan, 1-Brom-4-chlorbutan, 1-Brom-5-chlorpentan, 2,3-Dimethyl-3-brompentan, 3-Ethyl-3-brompentan, oder 4-Methyl-3-bromheptan.

Als Cycloalkylierungsmittel sei insbesondere Cyclopentylchlorid, Cyclohexylchlorid oder Cyclohexylbromid genannt.

Die veretherten Fluoresceinverbindungen der Formeln (1) bis (3) sind normalerweise farblos, jedoch teilweise auch schwach gefärbt. Wenn diese Farbbildner mit einem vorzugsweise sauren Entwickler, d.h. einem Elektronenakzeptor, in Kontakt gebracht werden, so ergeben sie je nach dem verwendeten Entwickler sofort intensive gelbe, orange oder rosarote Farbtöne, die besonders licht- und sublimationsecht sind.

Die Laktonverbindungen der Formeln (1) bis (3) sind auch sehr wertvoll im Gemisch mit einem oder mehreren anderen bekannten Farbbildnern, z.B. 3,3-(Bis-aminophenyl-)-phthaliden, 3-Indolyl-3-aminophenylazaphthaliden, (3,3-Bis-indolyl-)-phthaliden, 3-Amino-fluoranen, 3-Dialkylamino-7-di-benzylaminofluoranen, 3-Dialkylamino-6-methyl-7-arylaminofluoranen, Leukoauraminen, Spiropyranen, Spirodipyranen, Chromenoindolen, Phenoxazinen, Phenothiazinen, Chinazolinen, Rhodaminlaktamen, Carbazolylmethanen oder weiteren Triarylmethan-leukofarbstoffen, um graue oder schwarze Färbungen zu erzeugen.

Die Laktonverbindungen der Formeln (1) bis (3) zeigen sowohl auf aktivierten Tonen, wie auch auf phenolischen Unterlagen eine ausgezeichnete Farbintensität. Sie eignen sich insbesondere als sich schnell entwickelnde Farbbildner für die Verwendung in einem wärmeempfindlichen oder insbesondere druckempfindlichen Aufzeichnungsmaterial, das sowohl Kopierals auch Registriermaterial sein kann. Sie zeichnen sich dadurch aus, dass sie pH stabil, lichtecht und in den Kapselölen gut löslich sind. Nach Belichtung in CB-Blatt weisen sie eine geringe Abnahme der Farbstärke (CB-Desaktivierung) auf.

Ein druckempfindliches Material besteht beispielsweise aus mindestens einem Paar von Blättern, die mindestens einen Farbbildner der Formeln (1) bis (3) gelöst in einem organischen Lösungsmittel und einen Elektronenakzeptor als Entwickler enthalten.

Typische Beispiele für solche Entwickler sind Aktivton-Substanzen, wie Attapulgus-Ton, Säureton, Bentonit, Montmorillonit, aktivierter Ton, wie z.B. säureaktiviertes Bentonit oder Montmorillonit, ferner Zeolith, Halloysit, Siliciumdioxyd, Aluminiumoxid, Aluminiumsulfat, Aluminiumphosphat, Zirkondioxid, Zinkchlorid, Zinknitrat, aktiviertes Kaolin oder irgendein beliebiger Ton. Als Entwickler können auch sauer reagierende, organische Verbindungen, wie z.B. gegebenenfalls ringsubstituierte Phenole, Resorcine, Salicylsäuren, wie z.B. 3,5-Bis-(α,α-dimethylbenzyl-)-salicylsäure oder 3,5-Bis-(α-methylbenzyl)-salicylsäure oder Salicylsäureester und deren Metallsalze, z.B. Zinksalze, sowie ein sauer reagierendes, polymeres Material, wie z.B. ein phenolisches Polymerisat, ein Alkylphenolacetylenharz, ein Maleinsäure-Kolophonium-Harz oder ein teilweise oder vollständig hydrolysiertes Polymerisat von Maleinsäureanhydrid mit Styrol, Ethylen oder Vinylmethylether, oder Carboxymethylen verwendet werden. Es können auch Mischungen der genannten monomeren und polymeren Verbindungen eingesetzt werden. Besonders bevorzugte Entwickler sind säureaktiviertes Bentonit, Zinksalicylate oder die Kondensationsprodukte von p-substituierten Phenolen mit Formaldehyd. Die letzteren können auch mit Zink modifiziert sein.

Die Entwickler können zusätzlich auch im Gemisch mit an sich unreaktiven oder wenig reaktiven Pigmenten oder weiteren Hilfstoffen wie Kieselgel oder UV-Absorbern, wie z.B. 2-(2'-Hydroxyphenyl-)benztriazolen eingesetzt werden. Beispiele für solche Pigmente sind: Talk, Titandioxid, Aluminiumoxid, Aluminiumhydroxyd, Zinkoxid, Kreide, Tone wie Kaolin, sowie organische Pigmente, z.B. Harnstoff-Formaldehydkondensate (BET-Oberfläche 2-75 m²/g) oder Melamin-Formaldehyd-Kondensationsprodukte.

Der Farbbildner liefert an den Punkten, an denen er mit dem Elektronenakzeptor in Kontakt kommt, eine gefärbte Markierung. Um eine frühzeitige Aktivierung der in dem druckempfindlichen Aufzeichnungsmaterial vorhandenen Farbbildner zu verhindern, werden diese in der Regel von dem Elektronenakzeptor getrennt. Dies kann zweckmässig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen.

Beim Zerbrechen der Kapseln durch Druck, beispielsweise mittels eines Bleistiftes, wird die Farbbildnerlösung auf ein benachbartes mit einem Elektronenakzeptor beschichtetes Blatt übertragen, wodurch eine farbige Stelle erzeugt wird. Die Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z.B. polyhalogeniertes Paraffin oder Diphenyl, wie Chlorparaffin, Monochlordiphenyl oder Trichlordiphenyl, ferner Tricresylphosphat, Di-n-butylphthalat, Di-octylphthalat, Trichlorbenzol, Trichlorethylphosphat, aromatische Ether, wie Benzylphenylether, Kohlenwasserstofföle, wie Paraffin oder Kerosin, z.B. mit Isopropyl, Isobutyl, sek-Butyl oder tert.-Butyl alkylierte Derivate von Diphenyl, Naphthalin oder Terphenyl, Dibenzyltoluol, partiell hydriertes Terphenyl, mono- bis-,tetra-C₁-C₃-alkylierte Diphenylalkane, Dodecylbenzol, benzylierte Xylole, oder weitere chlorierte oder hydrierte, kondensierte, aromatische Kohlenwasserstoffe. Oft werden Mischungen verschiedener Lösungsmittel, insbesondere Mischungen aus Paraffinölen oder Kerosin und Diisopropylnaphthalin oder partiell hydriertem Terphenyl, eingesetzt, um eine optimale Löslichkeit für die Farbbildung, eine rasche und intensive Färbung und eine für die Mikroverkapselung günstige Viskosität zu erreichen.

Die Kapselwände können durch Koazervationskräfte gleichmässig um die Tröpfchen der Farbbildnerlösung herum gebildet werden, wobei das Einkapselungsmaterial z.B. in der US-Patentschrift 2,800,457 beschrieben ist. Die Kapseln können vorzugsweise auch aus einem Aminoplast oder modifizierten Aminoplasten durch Polykondensation gebildet werden, wie es in den britischen Patentschriften 989,264, 1,156,725, 1,301,052 und 1,355,124 beschrieben ist. Ebenfalls geeignet sind Mikrokapseln, welche durch Grenzflächenpolymerisation gebildet werden, wie z.B. Kapseln aus Polyester, Polycarbonat, Polysulfonamid, Polysulfonat, besonders aber aus Polyamid oder Polyurethan.

Die Farbbildner der Formeln (1) bis (3) enthaltenden Mikrokapseln können zur Herstellung von druckempfindlichen Kopiermaterialien der vershiedensten bekannten Arten verwendet werden. Die verschiedenen Systeme unterscheiden sich im wesentlichen voneinander durch die Anordnung der Kapseln, der Farbreaktanten und durch das Trägermaterial.

Bevorzugt wird eine Anordnung, bei der der eingekapselte Farbbildner in Form einer Schicht auf der Rückseite eines Uebertragungsblattes und der Elektronenakzeptor in Form einer Schicht auf der Vorderseite eines Empfangsblattes vorhanden sind.

Eine andere Anordnung der Bestandteile besteht darin, dass die den Farbbildner enthaltenden Mikrokapseln und der Entwickler in oder auf dem gleichen Blatt in Form einer oder mehrerer Einzelschichten oder in der Papierpulpe vorliegen.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel, wie Gummi arabicum, Polyvinylalkohol, Hydroxymethylcellulose, Casein, Methylcellulose, Dextrin, Stärke, Stärkederivate oder Polymerlatices. Letztere sind beispielsweise Butadien-Styrolcopolymerisate oder Acrylhomo- oder -copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet.

Die Verbindungen der Formeln (1) bis (3) können auch als Farbbildner in einem thermoreaktiven Aufzeichnungsmaterial verwendet werden. Dieses enthält in der Regel mindestens einen Schichtträger, einen Farbbildner, einen Elektronenakzeptor und gegebenenfalls auch ein Bindemittel und/oder Wachs. Gewünschtenfalls können auch Aktivatoren oder Sensibilisatoren im Aufzeichnungsmaterial vorhanden sein.

Thermoreaktive Aufzeichnungssysteme umfassen, z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten, wie z.B. Elektrocardiographen, verwendet. Die Bilderzeugung (Markierung) kann auch manuell mit einer erhitzten Feder erfolgen. Eine weitere Einrichtung zur Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst und dispergiert ist. Eine andere Möglichkeit besteht darin, dass sowohl der Farbbildner als auch der Entwickler in einer Schicht dispergiert sind. Das Bindemittel wird in spezifischen Bezirken mittels Wärme erweicht und an diesen Punkten, an denen Wärme angewendet wird, kommt der Farbbildner mit dem Elektronenakzeptor in Kontakt und es entwickelt sich sofort die erwünschte Farbe.

Als Entwickler eignen sich die gleichen Elektronenakzeptoren, wie sie in druckempfindlichen Papieren verwendet werden. Beispiele für Entwickler sind die bereits erwähnten Tonminerale und Phenolharze, oder auch phenolische Verbindungen, wie sie beispielsweise in der DE-PS 12,51,348 beschrieben sind, z.B. 4-tert.-Butylphenol, 4-Phenylphenol, Methylen-bis-(p-phenylphenol), 4-Hydroxydiphenylether, α-Naphthol, β-Naphthol, 4-Hydroxybenzoesäure-methylester oder -benzylester, 4-Hydroxydiphenylsulfon, 2,4-Dihydroxydiphenylsulfon, 4'-Hydroxy-4-methyldiphenylsulfon, 4'-Hydroxy-4-isopropoxydiphenylsulfon, 4-Hydroxy-acetophenon, 2,2'-Di-hydroxydiphenyl, 4,4'-Cyclohexylidendiphenol, 4,4'-Isopropylidendiphenol, 4,4'-Isopropyliden-bis-(2-methylphenol), ein Antipyrinkomplex von Zinkthiocyanat, ein Pyridinkomplex von Zinkthiocyanat, 4,4-Bis-(4-hydroxyphenyl)valeriansäure, Hydrochinon, Pyrogallol, Phloroglucin, p-, m-, o-Hydroxybenzoesäure, Gallussäure, 1-Hydroxy-2-naphthoesäure sowie Borsäure oder organische, vorzugsweise aliphatische Dicarbonsäuren, wie z.B. Weinsäure, Oxalsäure, Maleinsäure, Zitronensäure, Citraconsäure oder Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Laktonverbindungen und der Entwickler in Wasser schwer löslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so dass der Farbbildner mit dem Entwickler in Kontakt kommt und sich eine Farbe bilden kann. Wasserlösliche oder mindestens in Wasser quellbare Bindemittel sind z.B. hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, carboxylierte Butadien-Styrolcopolymerisate, Gelatine, Stärke oder veretherte Maisstärke.

Wenn der Farbbildner und der Entwickler in zwei getrennten Schichten vorliegen, können in Wasser unlösliche Bindemittel, d.h. in nichtpolaren oder nur schwach polaren Lösungsmitteln lösliche Bindemittel, wie z.B. Naturkautschuk, synthetischer Kautschuk, chlorierter Kautschuk, Polystyrol, Styrol/Butadien-Mischpolymerisate, Polymethylacrylate, Ethylcellulose, Nitrocellulose und Polyvinylcarbazol, verwendet werden. Die bevorzugte Anordnung ist jedoch diejenige, bei der der Farbbildner und der Entwickler in einer Schicht in einem wasserlöslichen Bindemittel enthalten sind.

Die thermoreaktiven Schichten können weitere Zusätze enthalten. Zur Verbesserung des Weissgrades, zur Erleichterung des Bedruckens der Papiere und zur Verhinderung des Festklebens der erhitzten Feder können diese Schichten, z.B. Talk, Titandioxyd, Zinkoxyd, Aluminiumhydroxyd, Calciumcarbonat (z.B. Kreide), Tone oder auch organische Pigmente, wie z.B. Harnstoff-Formaldehydpolymerisate enthalten. Um zu bewirken, dass nur innnerhalb eines begrenzten Temperaturbereiches die Farbe gebildet wird, können Substanzen, wie Harnstoff, Thioharnstoff, Diphenylthioharnstoff, Acetamid, Acetanilid, Benzolsulfanilid, Bis-Stearoyl-ethylendiamid, Stearinsäureamid, Phthalsäureanhydrid, Metallstearate, wie z.B. Zinkstearat, Phthalsäurenitril, Dimethylterephthalat oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse, z.B. Carnaubawachs, Montanawachs, Paraffinwachs, Polyethylenwachs, Kondensate höherer Fettsäureamide und Formaldehyde und Kondensate höherer Fettsäuren und Ethylendiamin.

Eine weitere Anwendung der Verbindungen der Formeln (1) bis (3) ist die Herstellung eines Farbbildes mittels photohärtbarer Mikrokapseln, wie sie z.B. in der DE-OS 3 247 488 beschrieben sind.

In den folgenden Beispielen beziehen sich die angegebenen Prozentsätze, wenn nichts anderes angegeben ist, auf das Gewicht. Teile bedeuten Gewichtsteile.

Beispiel 1: 54,3 g Kaliumhydroxid und 54,7 g Fluorescein werden nacheinander in 110 g Wasser gelöst. Anschliessend werden auch 149,2 g Isopentylbromid und 3 g Tetrabutylammoniumbromid darin gelöst. Diese Lösung wird auf Rückflusstemperatur aufgeheizt und 22 Stunden bei Rückfluss gehalten. Alsdann wird das Reaktionsgemisch mit 200 g Toluol und 80 g Wasser versetzt und 30 Minuten auf Rückflusstemperatur gehalten. Die Phasen werden getrennt. Die Toluolphase wird mit Wasser gewaschen und mit Natriumsulfat getrocknet, worauf das Toluol unter vermindertem Druck abdestilliert wird.

Nach Umkristallisation aus Isopropanol erhält man 41,1 g der Fluoranverbindung der Formel 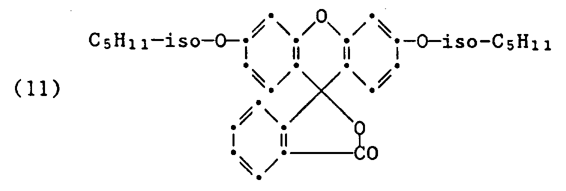
in Form von weissen Kristallen. Der Schmelzpunkt ist 126 - 128°C. Auf Säureton erzeugt diese Fluoranverbindung sofort eine intensive und lichtechte gelbe Färbung.

Beispiel 2: Verwendet man in Beispiel 1 anstelle von Isopentylbromid die äquivalente Menge von sek. Butylbromid und verfährt im übrigen wie in Beispiel 1 beschrieben, so erhält man eine Fluoranverbindung der Formel 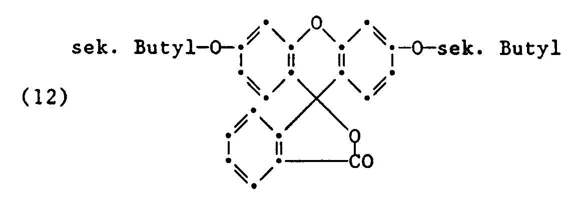
mit einem Schmelzpunkt von 65 - 70°C.

Auf Säureton erzeugt diese Fluoranverbindung sofort eine intensive gelbe Färbung.

### Beispiel 3:

(a) 11,2 g Kaliumhydroxid und 16,6 g Fluorescein werden nacheinander in 26 g Wasser gelöst. Hierauf werden 13,7 g n-Butylbromid und 0,3 g Tetrabutylammoniumbromid zugefügt. Die Lösung wird auf Rückflusstemperatur aufgeheizt und 2 Stunden bei dieser Temperatur gehalten. Die Isolierung des Reaktionsproduktes erfolgt wie in Beispiel 1 beschrieben. Man erhält 4,75 g einer orangen Verbindung der Formel 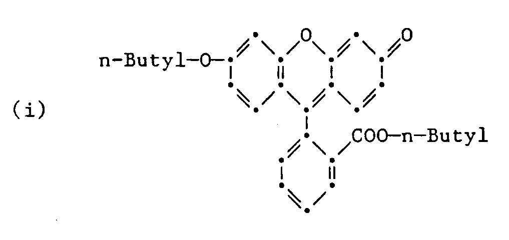 Nach Umkristallisation aus Isopropanol weist diese Verbindung einen Schmelzpunkt von 118 - 120°C auf.
(b) 4,3 g Kaliumhydroxid und 4,75 g der Verbindung der Formel (i) werden nacheinander in 7,3 g Wasser gelöst, worauf 8,6 g n-Octylbromid und 0,07 g Tetrabutylammoniumbromid hinzugefügt werden. Diese Lösung wird auf Rückflusstemperatur aufgeheizt und 22 Stunden bei dieser Temperatur gehalten. Alsdann wird das Reaktionsgemisch mit 200 g Toluol und 80 g Wasser versetzt und 30 Minuten auf Rückflusstemperatur gehalten. Die Phasen werden getrennt. Die Toluolphase wird mit Wasser gewaschen und mit Natriumsulfat getrocknet, worauf das Toluol unter vermindertem Druck abdestilliert wird. Man erhält 3,2 g einer unsymmetrisch substituierten Fluoranverbindung der Formel

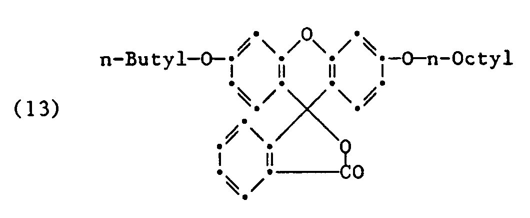

Beispiel 4: 22,4 g Kaliumhydroxid und 16,6 g Fluorescein werden nacheinander in 548 g Wasser gelöst. Anschliessend werden auch 12,3 g n-Propylbromid, 13,7 g n-Butylbromid und 0,3 g Tetrabutylammoniumbromid darin gelöst. Diese Lösung wird auf Rückflusstemperatur aufgeheizt und 22 Stunden bei Rückfluss gehalten. Alsdann wird das Reaktionsgemisch mit 200 g Toluol und 80 g Wasser versetzt und 30 Minuten auf Rückflusstemperatur gehalten. Die Phasen werden getrennt. Die Toluolphase wird mit Wasser gewaschen und mit Natriumsulfat getrocknet, worauf das Toluol unter vermindertem Druck abdestilliert wird.

Man erhält 11,5 g eines Gemisches, das nach HPLC-Analyse aus den Fluoranverbindungen
(a) 3,6-Di-n-propoxyfluoran
(b) 3-n-Butoxy-3-n-propoxyfluoran und
(c) 3,6-Di-n-butoxyfluoran
im Gewichtsverhältnis 1:2:1 ((a):(b):(c)) besteht.

Dieses Gemisch entwickelt auf Säureton eine intensive gelbe Farbe.

Beispiel 5: 98,2 g Kaliumhydroxid und 83 g Fluorescein werden nacheinander in 231 g Wasser gelöst. Anschliessend werden auch 226,6 g Isopentylbromid und 4,8 g Tetrabutylammoniumbromid darin gelöst. Diese Lösung wird auf Rückflusstemperatur aufgeheizt und 24 Stunden bei Rückfluss gehalten. Alsdann wird das Reaktionsgemisch mit 200 g Toluol und 80 g Wasser versetzt und 30 Minuten auf Rückflusstemperatur gehalten. Die Phasen werden getrennt. Die Toluolphase wird mit Wasser gewaschen und mit Natriumsulfat getrocknet, worauf das Toluol unter vermindertem Druck abdestilliert wird.

Nach Umkristallisation aus Isopropanol erhält man 58,7 g der Fluoranverbindung der Formel (11) in Form von weissen Kristallen. Der Schmelzpunkt ist 129 - 130°C. Auf Säureton erzeugt diese Fluoranverbindung sofort eine intensive und lichtechte gelbe Färbung.

Beispiel 6: 39,2 g Kaliumhydroxid und 33,2 g Fluorescein werden nacheinander in 92,4 g Wasser gelöst. Anschliessend werden auch 82,2 g sek.Butylbromid und 1,9 g Tetrabutylammoniumbromid darin gelöst. Diese Lösung wird auf Rückflusstemperatur aufgeheizt und 22 Stunden bei Rückfluss gehalten. Alsdann wird das Reaktionsgemisch mit 200 g Toluol und 80 g Wasser versetzt und 30 Minuten auf Rückflusstemperatur gehalten. Der Niederschlag wird abfiltriert. Nach Trocknen erhält man 20 g der Verbindung der Formel 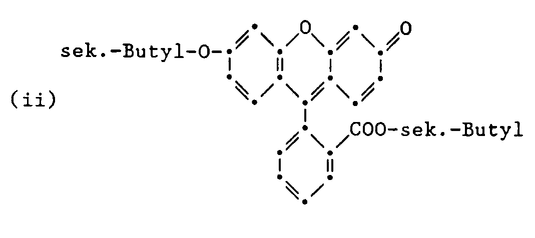
Smp. 134-137°C.

Die Phasen des Filtrates werden getrennt. Die Toluolphase wird mit Wasser gewaschen und mit Natriumsulfat getrocknet, worauf das Toluol unter vermindertem Druck abdestilliert wird.

Nach Umkristallisation aus Isopropanol erhält man 6,2 g der Fluoranverbindung der Formel (12) als amorphe, hellbraune Platten. Der Schmelzpunkt ist 50 - 60°C. Auf Säureton erzeugt diese Fluoranverbindung sofort eine gelbe Färbung.

Beispiel 7: 98,2 g Kaliumhydroxid und 83,0 g Fluorescein werden nacheinander in 110 g Wasser gelöst. Anschliessend werden auch 222,6 g n-Brompentan und 4,8 g Tetrabutylammoniumbromid darin gelöst. Diese Lösung wird auf Rückflusstemperatur aufgeheizt und 22 Stunden bei Rückfluss gehalten. Alsdann wird das Reaktionsgemisch mit 200 g Toluol und 80 g Wasser versetzt und 30 Minuten auf Rückflusstemperatur gehalten. Die Phasen werden getrennt. Die Toluolphase wird mit Wasser gewaschen und mit Natriumsulfat getrocknet, worauf das Toluol unter vermindertem Druck abdestilliert wird.

Nach Umkristallisation aus Isopropanol erhält man 46,0 g der Fluoranverbindung der Formel 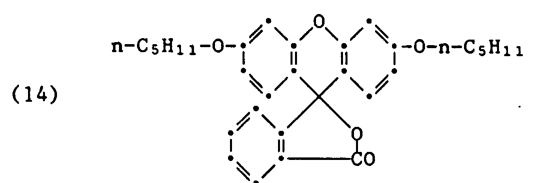
in Form von weissen Kristallen. Der Schmelzpunkt ist 112 - 113°C. Auf Säureton erzeugt diese Fluoranverbindung sofort eine intensive und lichtechte gelbe Färbung.

Beispiel 8: 5,8 g Kaliumhydroxid und 11,1 g der Verbindung der Formel (ii) werden nacheinander in 11,5 g Wasser gelöst. Anschliessend werden 11,9 g Isopentylbromid und 0,5 g Tetrabutylammoniumbromid hinzugefügt. Die Lösung wird auf Rückflusstemperatur aufgeheizt und 24 Stunden bei dieser Temperatur gehalten. Alsdann wird das Reaktionsgemisch mit 100 g Toluol und 40 g Wasser versetzt und 30 Minuten auf Rückflusstemperatur gehalten. Die Phasen werden getrennt. Die Toluolphase wird mit Wasser gewaschen und mit Natriumsulfat getrocknet, worauf das Toluol unter vermindertem Druck abdestilliert wird.

Nach Umkristallisation aus Isopropanol erhält man 4,9 g der unsymmetrisch substituierten Fluoranverbindung der Formel 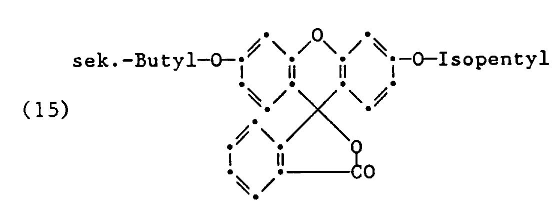
Beispiel 9: 115,5 g Kaliumhydroxid und 83 g Fluorescein werden nacheinander in 231 g Wasser gelöst. Anschliessend werden auch 92,2 g n-Propylbromid, 102,8 g n-Butylbromid und 4,8 g Tetrabutylammoniumbromid darin gelöst. Diese Lösung wird auf Rückflusstemperatur aufgeheizt und 22 Stunden bei Rückfluss gehalten. Alsdann wird das Reaktionsgemisch mit 200 g Toluol und 80 g Wasser versetzt und 30 Minuten auf Rückflusstemperatur gehalten. Die Phasen werden getrennt. Die Toluolphase wird mit Wasser gewaschen und mit Natriumsulfat getrocknet, worauf das Toluol unter vermindertem Druck abdestilliert wird.

Man erhält 127,15 g eines Gemisches, das nach HPLC-Analyse aus den Fluoranverbindungen
(a) 3,6-Di-n-propoxyfluoran
(b) 3-n-Butoxy-3-n-propoxyfluoran und
(c) 3,6-Di-n-butoxyfluoran
im Gewichtsverhältnis 1:2:1 ((a):(b):(c)) besteht.

Nach Reinigung weist das Gemisch 54,7 g weisse Kristalle mit einem Schmelzpunkt von 103-105°C auf.

Dieses Gemisch entwickelt auf Säureton eine intensive gelbe Farbe.

### Beispiel 10:

a) 49,8 g Fluorescein, 18,6 g Dimethylmethanphosphonat und 34,55 g Kaliumcarbonat werden 3 Stunden auf 100°C erwärmt. Alsdann werden 300 ml Wasser zugegeben. Hierauf wird der Niederschlag abfiltriert und getrocknet. Man erhält 51,3 g einer Verbindung der Formel 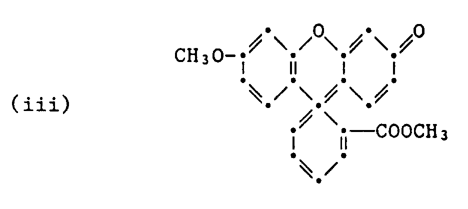 Smp. 207-211°C.
(b) 32,89 g Kaliumhydroxid und 51,3 g der Verbindung der Formel (iii) werden nacheinander in 65,8 g Wasser gelöst. Anschliessend werden 58,5 g n-Butylbromid und 2,75 g Tetrabutylammoniumbromid darin gelöst.
   Diese Lösung wird auf Rückflusstemperatur aufgeheizt und 24 Stunden bei Rückfluss gehalten. Alsdann wird das Reaktionsgemisch mit 200 g Toluol und 80 g Wasser versetzt und 30 Minuten auf Rückflusstemperatur gehalten. Die Phasen werden getrennt. Die Toluolphase wird mit Wasser gewaschen und mit Natriumsulfat getrocknet, worauf das Toluol unter vermindertem Druck abdestilliert wird.
   Nach Umkristallisation aus Isopropanol erhält man 23,7 g der Fluoranverbindung der Formel 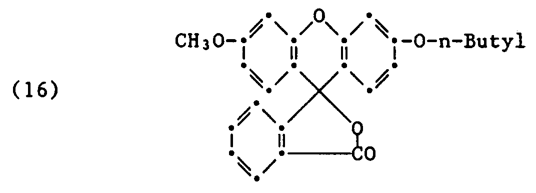 in Form von hellgelben Kristallen. Der Schmelzpunkt ist 129-131°C.

### Beispiel 11:

a) 3,32 g Fluorescein, 2,74 g N-Butylbromid, 8,3 g Kaliumcarbonat, 50 ml Aceton und 0,19 g Tetrabutylammoniumbromid werden 17 Stunden am Rückfluss erhitzt, worauf der Rückstand abfiltriert und Aceton entfernt wird. Man erhält 2,74 g der Verbindung der Formel (i), die nach Behandlung mit Hexan einen Schmelzpunkt von 120-124°C aufweist.
b) 11,1 g der gemäss (a) hergestellten Verbindung der Formel (i), 5,78 g Kaliumhydroxid werden nacheinander in 11,55 g Wasser gelöst. Anschliessend werden 11,3 g n-Pentylbromid und 0,48 g Tetrabutylammoniumbromid darin gelöst.
   Diese Lösung wird auf Rückflusstemperatur aufgeheizt und 20 Stunden bei Rückfluss gehalten. Alsdann wird das Reaktionsgemisch mit 100 g Toluol und 40 g Wasser versetzt und 30 Minuten auf Rückflusstemperatur gehalten. Die Phasen werden getrennt. Die Toluolphase wird mit Wasser gewaschen und mit Natriumsulfat getrocknet, worauf das Toluol unter vermindertem Druck abdestilliert wird.
   Nach Umkristallisation aus Isopropanol erhält man 6,1 g der Fluoranverbindung der Formel 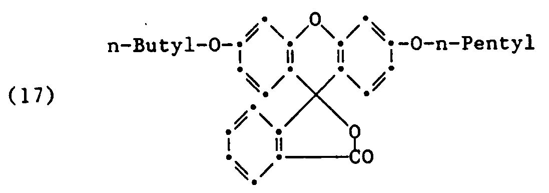 Der Schmelzpunkt ist 108-109°C.

Beispiel 12: 11,1 g der gemäss Beispiel 11(a) hergestellten Verbindung der Formel (i) und 5,78 g Kaliumhydroxid werden nacheinander in 11,55 g Wasser gelöst. Anschliessend werden 11,92 g 2-Pentylbromid und 0,48 g Tetrabutylammoniumbromid darin gelöst. Diese Lösung wird auf Rückflusstemperatur aufgeheizt und 20 Stunden bei Rückfluss gehalten. Alsdann wird das Reaktionsgemisch mit 100 g Toluol und 40 g Wasser versetzt und 30 Minuten auf Rückflusstemperatur gehalten. Die Phasen werden getrennt. Die Toluolphase wird mit Wasser gewaschen und mit Natriumsulfat getrocknet, worauf das Toluol unter vermindertem Druck abdestilliert wird.

Nach Umkristallisation aus Isopropanol erhält man 3,47 g der Fluoranverbindung der Formel 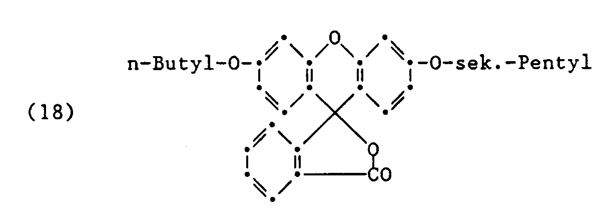
Der Schmelzpunkt ist 47-51°C.

### Beispiel 13: Herstellung eines druckempfindlichen Kopierpapiers

Eine Lösung von 3 g der Laktonverbindung der Formel (11) in 80 g Di-Isopropylnaphthalin und 17 g Kerosin wird auf an sich bekannte Weise mit Gelatine und Gummi arabicum durch Koazervation mikroverkapselt, mit Stärkelösung vermischt und auf ein Blatt Papier gestrichen. Ein zweites Blatt Papier wird auf der Frontseite mit säureaktiviertem Bentonit als Farbentwickler beschichtet. Das erste, den Farbbildner enthaltende Blatt und das mit Farbentwickler beschichtete Papier werden mit den Beschichtungen benachbart aufeinandergelegt. Durch Schreiben mit der Hand oder mit der Schreibmaschine auf dem ersten Blatt wird Druck ausgeübt, und es entwickelt sich sofort auf dem mit dem Entwickler beschichteten Blatt eine intensive gelbe Kopie, die ausgezeichnet sublimier- und lichtecht ist.

Entsprechende intensive, sublimier- und lichtechte Kopien werden auch bei Verwendung der Farbbildner gemäss den Beispielen 2 bis 12 erzielt.

Beispiel 14: Ersetzt man in Beispiel 13 die Laktonverbindung der Formel (11) durch eine Mischung der folgenden Zusammensetzung
1,2 g 3,3-Bis-(4'-dimethylaminophenyl)-6-dimethylaminophthalid,
1,2 g N-Butylcarbazol-3-yl-bis-(4'-N-methyl-N-phenylaminophenyl)-methan
1,2 g der Laktonverbindung der Formel (11) und
0,4 g 3,3-Bis-(N-octyl-2'methylindol-3'-yl-)phthalid
und verfährt im übrigen wie in Beispiel 13 beschrieben, so erhält man ein druckempfindliches Aufzeichnungsmaterial, welches durch Schreiben mit der Hand oder mit der Schreibmaschine eine intensive und lichtechte schwarze Kopie ergibt.

Beispiel 15: 1 g der Laktonverbindung der Formel (12) gemäss Beispiel 2 wird in 17 g Toluol gelöst. Zu dieser Lösung gibt man unter Rühren 12 g Polyvinylacetat, 8 g Calciumcarbonat und 2 g Titandioxyd. Die erhaltene Suspension wird im Gewichtsverhältnis 1:1 mit Toluol verdünnt und mit einem 10 »m Rakel auf ein Blatt Papier gestrichen. Auf dieses Blatt Papier wird ein zweites Blatt Papier gelegt, dessen Unterseite bei einem Auftragsgewicht von 3 g/m² mit einer Mischung bestehend aus 1 Teil eines Amidwachses, 1 Teil eines Stearinwachses und 1 Teil Zinkchlorid beschichtet worden ist. Durch Schreiben mit der Hand oder mit der Schreibmaschine auf dem oberen Blatt wird Druck ausgeübt, und es entwickelt sich sofort auf dem mit den Farbbildner beschichteten Blatt, eine intensive, sublimier- und lichtechte gelbe Farbe.

### Beispiel 16: Herstellung eines wärmeempfindlichen Aufzeichnungsmaterials

In einer Kugelmühle werden 32 g 4,4'-Isopropylidendiphenol (Bisphenol A), 3,8 g Distearylamid des Ethylendiamins, 39 g Kaolin, 20 g eines 88 % hydrolysierten Polyvinylalkohols und 500 ml Wasser gemahlen bis die Teilchengrösse ca 5 »m beträgt. In einer zweiten Kugelmühle werden 6 g der Laktonverbindung der Formel (11), 3 g eines zu 88 % hydrolysierten Polyvinylalkohols und 60 ml Wasser zu einer Teilchengrösse von ca. 3 »m gemahlen.

Die beiden Dispersionen werden zusammengegeben und mit einem Trockenauftragsgewicht von 5,5 g/m² auf ein Papier gestrichen. Durch Berührung des Papiers mit einem erhitzten Metallstift wird eine intensive orange Farbe erhalten, die eine ausgezeichnete Sublimier- und Lichtechtheit hat.

Intensive und lichtechte Farben werden auch bei Verwendung der Farbbildner gemäss den Beispielen 2 bis 12 erhalten.

## Patentansprüche

1. Veretherte Fluoresceinverbindungen der Formel 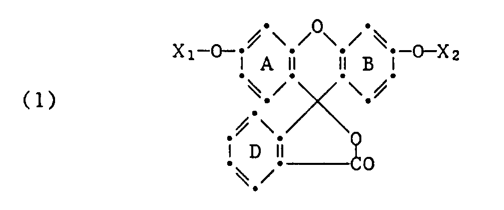 worin
X₁ Alkyl mit 4 bis 12 Kohlenstoffatomen oder C₅-C₆-Cycloalkyl und
X₂ unsubstituiertes oder durch Halogen, Hydroxy, Cyano oder C₁-C₅-Alkoxy substituiertes Alkyl mit 1 bis 12 Kohlenstoffatomen oder C₅-C₆-Cycloalkyl bedeuten, die Ringe A und B, unabhängig voneinander, unsubstituiert oder durch Halogen, C₁-C₅-Alkyl oder C₁-C₅-Alkoxy substituiert sind und der Ring D unsubstituiert oder durch Halogen, Nitro, C₁-C₅-Alkyl, C₁-C₅-Alkoxy, C₁-C₅-Alkylthio, C₁-C₅-Alkoxycarbonyl, Amino, Mono C₁-C₅-Alkylamino oder Di C₁-C₅-Alkylamino substituiert ist, mit der Massgabe, dass X₁ und X₂ nicht gleichzeitig n-Butyl darstellen.

2. Verbindungen gemäss Anspruch 1, dadurch gekennzeichnet, dass in Formel (1)
X₁ n-Butyl, Isobutyl, sek. Butyl, 1,1,3,3-Tetramethylbutyl, n-Pentyl, Isopentyl, n-Hexyl oder n-Octyl und
X₂ C₁-C₈-Alkyl, 2-Cyanoethyl, 2-Methoxyethyl oder 2-Ethoxyethyl, vorzugsweise Methyl, Ethyl, n-Propyl, Isopropyl, n-Butyl, sek. Butyl, 1,1,3,3-Tetramethylbutyl, Isopentyl oder n-Octyl bedeuten.

3. Verbindungen gemäss einem der Ansprüche 1 und 2, dadurch gekennzeichnet, das in Formel (1) die Ringe A und B, unabhängig voneinander, unsubstituiert oder jeweils durch 1 oder 2 Halogenatome substituiert sind.

4. Verbindungen gemäss einem der Ansprüche 1 bis 3, dadurch gekennzeichnet, dass in Formel (1) der Ring D unsubstituiert ist.

5. Verbindungen gemäss einem der Ansprüche 1 bis 4, dadurch gekennzeichnet, dass in Formel (1) die Ringe A und B unsubstituiert sind.

6. Verbindungen gemäss Anspruch 1, dadurch gekennzeichnet, dass sie der Formel 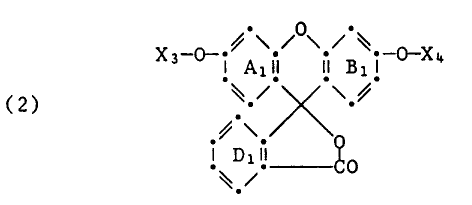 entsprechen, worin
X₃ C₄-C₈-Alkyl und
X₄ C₁-C₈-Alkyl oder durch Halogen, Cyano oder C₁-C₅-Alkoxy substituiertes C₂-C₄-Alkyl bedeuten und
die Ringe A₁, B₁ und D₁, unabhängig voneinander, unsubstituiert oder durch Halogen substituiert sind.

7. Verbindungen gemäss Anspruch 6, dadurch gekennzeichnet, dass in Formel (2)
X₄ C₁-C₅-Alkyl bedeutet, die Ringe A₁, B₁ und D₁ unsubstituiert sind.

8. Verbindungen gemäss Anspruch 1, dadurch gekennzeichnet, dass sie der Formel 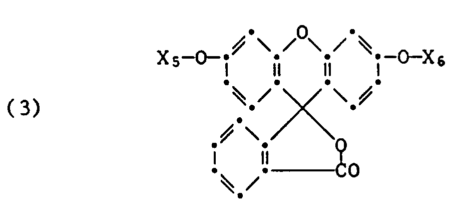 entsprechen, worin
X₅ n-Butyl, sek. Butyl, Isobutyl, n-Pentyl, Isopentyl, Hexyl oder n-Octyl und
X₆ Methyl, Ethyl, n-Propyl, Isopropyl, n-Butyl, sek. Butyl, n-Pentyl oder Isopentyl
bedeuten.

9. Verbindungen gemäss Anspruch 8, dadurch gekennzeichnet, dass in Formel (3) X₅ n-Octyl und X₆ n-Butyl oder X₅ n-Butyl oder sek.Butyl und X₆ n-Pentyl oder Isopentyl bedeuten.

10. Verbindungen gemäss einem der Ansprüche 1 bis 9, dadurch gekennzeichnet, dass sie in Form eines Gemisches vorliegen.

11. Farbbildnergemisch bestehend aus 3,6-Di-n-propoxyfluoran, 3-n-Butoxy-6-n-propoxy-fluoran und 3,6-Di-n-butoxyfluoran.

12. Verfahren zur Herstellung von veretherten Fluoresceinverbindungen der Formel (1) gemäss Anspruch 1, dadurch gekennzeichnet, dass man eine Fluoresceinverbindung der Formel 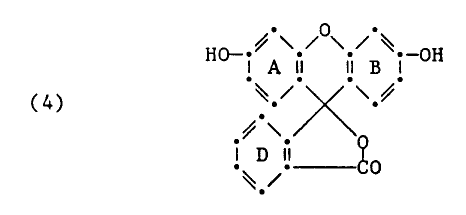 worin A, B und D die in Anspruch 1 angegebene Bedeutung haben, in beliebiger Reihenfolge mit entsprechenden Alkylierungsmitteln bzw. Cycloalkylierungsmitteln umsetzt.

13. Verwendung von Laktonverbindungen gemäss Ansprüchen 1 bis 11 als Farbbildner in einem druckempfindlichen oder wärmeempfindlichen Aufzeichnungsmaterial.

14. Druck- oder wärmeempfindliches Aufzeichnungsmaterial, dadurch gekennzeichnet, dass es in seinem Farbreaktantensystem als Farbbildner mindestens eine Laktonverbindung gemäss einem der Ansprüche 1 bis 11 enthält.

15. Druckempfindliches Aufzeichnungsmaterial gemäss Anspruch 14, dadurch gekennzeichnet, dass es die Laktonverbindung, gelöst in einem organischen Lösungsmittel, und mindestens einen festen Elektronenakzeptor enthält.

16. Druckempfindliches Aufzeichnungsmaterial gemäss einem der Ansprüche 14 und 15, dadurch gekennzeichnet, dass die Laktonverbindung in Mikrokapseln eingekapselt ist.

17. Druckempfindliches Aufzeichnungsmaterial gemäss Anspruch 16, dadurch gekennzeichnet, dass die eingekapselte Laktonverbindung in Form einer Schicht auf der Rückseite eines Uebertragungsblattes und der Elektronenakzeptor in Form einer Schicht auf der Vorderseite des Empfangsblattes vorhanden sind.

18. Wärmeempfindliches Aufzeichnungsmaterial gemäss Anspruch 17, dadurch gekennzeichnet, dass es in mindestens einer Schicht mindestens eine Laktonverbindung gemäss einem der Ansprüche 1 bis 11, einen Elektronenakzeptor und gegebenenfalls ein Bindemittel und/oder Wachs enthält.

19. Aufzeichnungsmaterial gemäss einem der Ansprüche 14 bis 18, dadurch gekennzeichnet, dass die Laktonverbindung gemeinsam mit einem oder mehreren anderen Farbbildnern enthalten ist.

## Claims

1. An etherified fluorescein compound of the formula 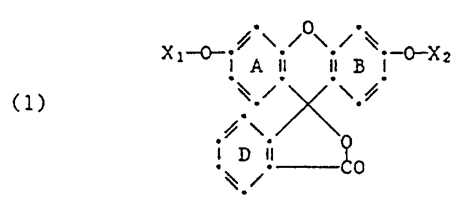 in which X₁ is alkyl having 4 to 12 carbon atoms or C₅-C₆cycloalkyl and X₂ is alkyl having 1 to 12 carbon atoms, which is unsubstituted or substituted by halogen, hydroxyl, cyano or C₁-C₅alkoxy, or C₅-C₆cycloalkyl, the rings A and B, independently of one another, are unsubstituted or substituted by halogen, C₁-C₅alkyl or C₁-C₅alkoxy, and the ring D is unsubstituted or substituted by halogen, nitro, C₁-C₅alkyl, C₁-C₅alkoxy, C₁-C₅alkylthio, C₁-C₅alkoxycarbonyl, amino, mono-C₁-C₅alkylamino or di-C₁-C₅alkylamino, with the proviso that X₁ and X₂ are not at the same time n-butyl.

2. A compound according to claim 1 wherein, in formula (1), X₁ is n-butyl, isobutyl, sec-butyl, 1,1,3,3-tetramethylbutyl, n-pentyl, isopentyl, n-hexyl or n-octyl and X₂ is C₁-C₈alkyl, 2-cyanoethyl, 2-methoxyethyl or 2-ethoxyethyl, preferably methyl, ethyl, n-propyl, isopropyl, n-butyl, sec-butyl, 1,1,3,3-tetramethylbutyl, isopentyl or n-octyl.

3. A compound according to any one of claims 1 and 2, wherein, in formula (1), the rings A and B, independently of one another, are unsubstituted or are each substituted by 1 or 2 halogen atoms.

4. A compound according to any one of claims 1 to 3, wherein, in formula (1), the ring D is unsubstituted.

5. A compound according to any one of claims 1 to 4, wherein, in formula (1), the rings A and B are unsubstituted.

6. A compound according to claim 1, which has the formula 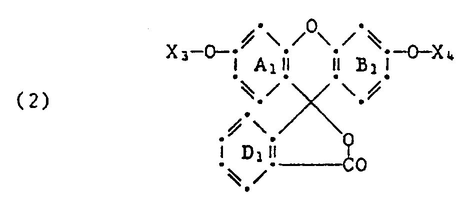 in which X₃ is C₄-C₈alkyl and X₄ is C₁-C₈alkyl, or C₂-C₄alkyl which is substituted by halogen, cyano or C₁-C₅alkoxy, and the rings A₁, B₁ and D₁, independently of one another, are unsubstituted or substituted by halogen.

7. A compound according to claim 6, wherein, in formula (2), X₄ is C₁-C₅alkyl and the rings A₁, B₁ and D₁ are unsubstituted.

8. A compound according to claim 1, which has the formula 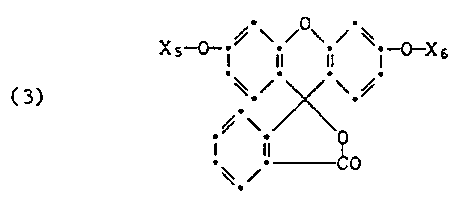 in which X₅ is n-butyl, sec-butyl, isobutyl, n-pentyl, isopentyl, hexyl or n-octyl and X₆ is methyl, ethyl, n-propyl, isopropyl, n-butyl, sec-butyl, n-pentyl or isopentyl.

9. A compound according to claim 8, wherein, in formula (3), X₅ is n-octyl and X₆ is n-butyl or X₅ is n-butyl or sec-butyl and X₆ is n-pentyl or isopentyl.

10. A compound according to any one of claims 1 to 9, which is in the form of a mixture.

11. A mixture of colour formers consisting of 3,6-di-n-propoxyfluorane, 3-n-butoxy-6-n-propoxyfluorane and 3,6-di-n-butoxyfluorane.

12. A process for the preparation of an etherified fluorescein compound of the formula (1) according to claim 1, which comprises reacting a fluorescein compound of the formula 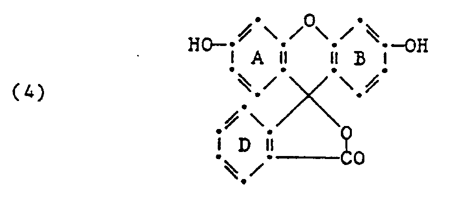 in which A, B and D are as defined in claim 1 with appropriate alkylating agents or cycloalkylating agents in any desired sequence.

13. Use of a lactone compound according to any one of claims 1 to 11 as colour former in a pressure-sensitive or heat-sensitive recording material.

14. A pressure-sensitive or heat-sensitive recording material which comprises, as colour former in its colour reactant system, at least one lactone compound according to any one of claims 1 to 11.

15. A pressure-sensitive recording material according to claim 14, wherein the lactone compound is dissolved in an organic solvent, and which recording material comprises at least one solid electron acceptor.

16. A pressure-sensitive recording material according to any one of claims 14 and 15, wherein the lactone compound is encapsulated in microcapsules.

17. A pressure-sensitive recording material according to claim 16, wherein the encapsulated lactone compound is present in the form of a layer on the reverse side of a transfer sheet and the electron acceptor is present in the form of a layer on the front side of the receiver sheet.

18. A heat-sensitive recording material according to claim 17, which comprises, in at least one layer, at least one lactone compound according to any one of claims 1 to 11, an electron acceptor and, optionally, a binder and/or wax.

19. A recording material according to any one of claims 14 to 18, wherein the lactone compound is present together with one or more other colour formers.

## Revendications

1. Composés éthérifiés de type fluorescéine de formule 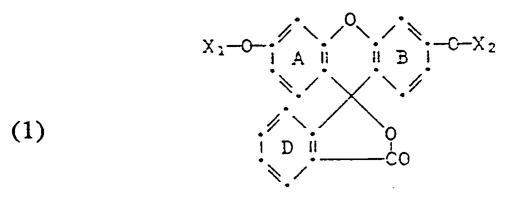 dans laquelle X₁ représente un groupe alkyle comportant 4 à 12 atomes de carbone ou un groupe cycloalkyle en C₅-C₆, et
X₂ représente un groupe alkyle non substitué ou substitué par un atome d'halogène, un groupe hydroxy, cyano ou alkoxy en C₁-C₅, comportant entre 1 et 12 atomes de carbone ou un groupe cycloalkyle en C₅-C₆, les noyaux A et B sont indépendamment non substitués ou substitués par un atome d'halogène, un groupe alkyl en C₁-C₅, ou un groupe alkoxy en C₁-C₅, et le noyau D est non substitué ou substitué par un atome d'halogène, un groupe nitro, alkyle en C₁-C₅, alkoxy en C₁-C₅, alkylthio en C₁-C₅, alkoxycarbonyle en C₁-C₅, amino, monoalkylamino en C₁-C₅, ou dialkyle(en C₁-C₅)amino, avec la réserve que X₁ et X₂ ne représentent pas tous les deux simultanément un groupe n-butyle.

2. Composés conformes à la revendication 1, caractérisés en ce que dans la formule (1)
X₁ représente un groupe n-butyle, isobutyle, sec-butyle, 1,1,3,3-tétraméthylbutyle, n-pentyle, isopentyle, n-hexyle ou n-octyle et
X₂ un groupe alkyle en C₁-C₈, 2-cyanoéthyle, 2-méthoxyéthyle, 2-éthoxyéthyle et de préférence un méthyle, un éthyle, un n-propyle, un isopropyle, un n-butyle, un sec-butyle, un 1,1,3,3-tétraméthylbutyle, un isopentyle ou un n-octyle.

3. Composés conformes à une des revendications 1 ou 2, caractérisés en ce que les noyaux A et B sont indépendàmment non substitués ou substitués chacun par 1 ou 2 atomes d'halogène.

4. Composés conformes à une des revendications 1 à 3, caractérisés en ce que, dans la formule (1), le noyau D n'est pas substitué.

5. Composés conformes à une des revendications 1 à 4, caractérisés en ce que, dans la formule (1), les noyaux A et B ne sont pas substitués.

6. Composés conformes à la revendication 1, caractérisés en ce qu'ils correspondent à la formule 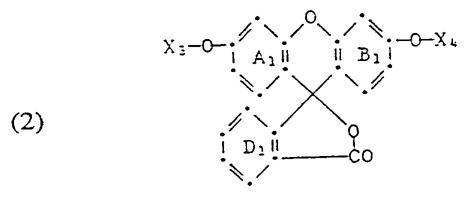 dans laquelle
X₃ représente un groupe alkyle en C₄-C₈,
X₄ représente un groupe alkyle en C₁-C₈ ou un groupe alkyle en C₂-C₄ substitué par un atome d'halogène un groupe cyano ou alkoxy en C₁-C₅, et les noyaux A₁, B₁ et D₁, sont indépendamment non substitués ou halogénés.

7. Composés conformes à la revendication 6, caractérisés en ce que, dans la formule (2), X₄ représente un groupe alkyle en C₁-C₅, et les noyaux A₁, B₁ et D₁ ne sont pas substitués.

8. Composés conformes a la revendication 1, caractérisés en ce qu'ils correspondent à la formule 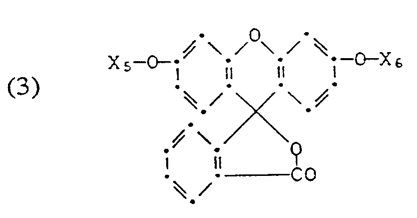 dans laquelle
X₅ représente un groupe n-butyle, sec-butyle, isobutyle, n-pentyle, isopentyle, hexyle ou n-octyle et
X₆ représente un groupe méthyle, éthyle, n-propyle, isopropyle, n-butyle, sec-butyle, n-pentyle ou isopentyle.

9. Composés conformes à la revendication 8, caractérisés en ce que dans la formule (3), X₅ représente un groupe n-octyle et X₆ un groupe n-butyle ou X₅ un groupe n-butyle ou sec-butyle et X₆ un groupe n-pentyle ou isopentyle.

10. Composés conformes à une des revendications 1 à 9, caractérisés en ce qu'ils se trouvent sous forme de mélanges.

11. Mélange d'agents chromogènes, composé de 3,6-di-n-propoxyfluoranne, de 3-n-butoxy-6-n-propoxyfluoranne et de 3,6-di-n-butoxyfluoranne.

12. Procédé de préparation de composés éthérifiés de fluorescéine de formule (1) conformes à la revendication 1, caractérisé en ce que l'on fait réagir un composé de fluorescéine de formule 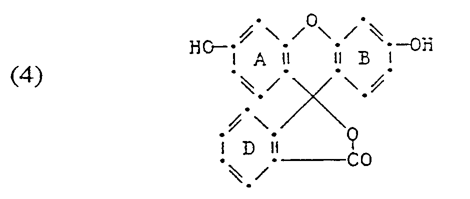 dans laquelle A, B et D sont ceux définis dans la revendication 1, dans un ordre quelconque, avec les agents d'alkylation ou de cycloalkylation correspondants.

13. Utilisation des composés de type lactone conformes à une des revendications 1 à 11 comme agents chromogènes dans un matériau d'enregistrement sensible à la pression ou à la chaleur.

14. Matériau d'enregistrement sensible à la pression ou à la chaleur, caractérisé en ce qu'il contient dans son système de réactifs de couleur au moins un composé de type lactone conforme à une des revendications 1 à 11.

15. Matériau d'enregistrement sensible à la pression conforme à la revendication 14, caractérisé en ce qu'il contient le composé de type lactone, dissous dans un solvant organique, et au moins un accepteur d'électrons solide.

16. Matériau d'enregistrement sensible à la pression, conforme à une des revendications 14 et 15, caractérisé en ce que le composé de type lactone est encapsulé dans des microcapsules.

17. Matériau d'enregistrement sensible à la pression, conforme à la revendication 16, caractérisé en ce que composé de type lactone encapsulé se trouve sous forme d'une couche sur le côté verso d'une feuille de transfert et l'accepteur d'électrons sous forme d'une couche sur le côté recto d'une feuille réceptrice.

18. Matériau d'enregistrement conforme à la revendication 17, caractérisé en ce qu'il contient au moins dans une couche, au moins un composé de type lactone conforme à une des revendications 1 à 11, un accepteur d'électrons et éventuellement un liant et/ou une cire.

19. Matériau conforme à une des revendications 14 à 18, caractérisé en ce que le composé de type lactone est présent ensemble avec un ou plusieurs autres agents chromogènes.
